Europäisches Patentamt

European Patent Office

Office européen des brevets

(11) Publication number: **0 418 384 A1**

## EUROPEAN PATENT APPLICATION
### published in accordance with Art. 158(3) EPC

(21) Application number: 89905167.6

(22) Date of filing: 09.05.89

(86) International application number:
PCT/JP89/00476

(87) International publication number:
WO 89/11096 (16.11.89 89/27)

(51) Int. Cl.⁵: **G01N 27/30**

(30) Priority: 09.05.88 JP 110448/88

(43) Date of publication of application:
27.03.91 Bulletin 91/13

(84) Designated Contracting States:
**BE DE FR GB**

(71) Applicant: **TERUMO KABUSHIKI KAISHA**
**No. 44-1, Hatagaya 2-chome, Shibuya-ku**
**Tokyo 151(JP)**

(72) Inventor: **SHIMOMURA, Takeshi**
**Terumo Kabushiki Kaisha 2656-1, Ohbuchi**
**Fuji-shi Shizuoka 417(JP)**
Inventor: **KATSUBE, Teruaki**
**1-1-205, Nagahama 3-chome**
**Tama-shi Tokyo 206(JP)**
Inventor: **OYAMA, Noboru**
**5-24, Shinmachi 3-chome**
**Fuchu-shi Tokyo 183(JP)**

(74) Representative: **Gillard, Marie-Louise et al**
**Cabinet Beau de Loménie 55, Rue**
**d'Amsterdam**
**F-75008 Paris(FR)**

(54) **ENZYME SENSOR.**

(57) This invention relates to an enzyme sensor consisting of a conductive layer (2), and an enzyme immobilization layer(3) formed so as to cover the conductive layer (2) therewith, characterized in that at least apart of the outer surface of the enzyme immobilization layer (3) is provided with grooves (5). This enzyme sensor is capable of preventing the leakage of enzyme, and measuring the concentration of an organic matrix with a high accuracy using a minute and highly-sensitive structure.

F I G. 4

## ENZYME SENSOR

### BACKGROUND ART

The present invention relates to an enzyme sensor which measures the concentrations of biosubstrates by enzymatic action.

Hitherto, enzymes have been fixed by using a cross linking agent such as glutaraldehyde to coat an enzyme fixed layer on the sensitive part of the electrode. However, in this method, a certain measure of electrode space is required. Recently, although various methods for providing an enzyme sensor with an improved layer coating have been suggested, an enzyme sensor which can prevent leakage of enzymes and measure with high accuracy has not been provided. As for the coating method for the enzyme fixed layer, only a lift-off method making a pool at the gate part of the semiconductor substrate and injecting the aforementioned glutaraldehyde and the enzyme into the pool for improving the sensor in the semiconductor technique is disclosed.

### DISCLOSURE OF INVENTION

The purpose of this invention is to solve the problems of the above-described conventional art and provide an enzyme sensor which can prevent the elusion of enzyme and measure the concentration of the biosubstrate with high sensitivity and high accuracy in spite of the sensor's compactness.

To solve these problems, the enzyme sensor of the present invention is provided with an electrically conductive layer and an enzyme fixed layer which fixes the enzyme coating the electrically conductive layer. A groove part is formed on at least some portion of the surface of the enzyme fixed layer. The electrically conductive layer is preferably selected from iridium oxide, palladium oxide or ruthenium oxide.

As a results of the above improvements, an enzyme sensor which can prevent elusion of enzymes and measure a concentration of the biosubstrate with high sensitivity and high accuracy in spite of its compactness is provided by the present invention.

Furthermore, the other purposes and effects of the present invention will be clarified by referring to the following detailed explanation of the embodiment and the accompanying figures.

### BRIEF DESCRIPTION OF DRAWINGS

Fig. 1A shows a front elevational view of a typical enzyme sensor of the first embodiment.

Fig. 1B shows an on line AA' cross-sectional view of a typical enzyme sensor of the first embodiment.

Fig. 2 shows a cyclic voltamogram of the electrolytic reaction of the first embodiment.

Fig. 3 shows the relationship between the thickness of the enzyme fixed layer and the output voltage of the enzyme sensor of the first embodiment.

Fig. 4 shows an enlarged cross-sectional view of the sensitive part of the enzyme sensor of the second and third embodiments.

Fig. 5 shows the output voltage of the enzyme sensor of the second and third embodiments.

### BEST MODE FOR CARRYING OUT THE INVENTION

In the enzyme sensor of the present invention, an FET is used to raise the sensing speed and a layer comprising an electrolytically polymerized layer as the enzyme carrying layer carrying the enzymes (glucose oxydase, urease and so on) is formed after being coated with a layer of electrically conductive substances (iridium oxide: $IrO_x$, palladium oxide: $PdO_x$, ruthenium oxide: $RuO_x$) on the isolation gate part of this FET.

Although a MOS-type FET can be used as the FET, a junction-type FET is preferable since the gate channel part can be sufficiently protected from the subject solution in case the isolation gate part is used. Since a gate insulating layer is not used in the junction-type FET, this type of enzyme sensor is resistant to impulse voltage and simple to manufacture, and therefore the junction-type FET has good reliability. Furthermore, it is suitable for high sensitization because it has a low noise level which noise level is induced by the low frequency.

2

As for the substance forming the electrolytically polymerized layer, pyrrole, pyrrole derivatives, oxygen ring compounds having a pyrrole nucleus, phenylenediamine, phenol, thiophene and thiophene derivatives shall preferably be used.

The present invention has an enzyme sensor coating with an enzyme fixed layer (electrolytic layer) on the surface layer of the $IrO_x$ on the isolation gate of the FET. Further, the present invention provides a compact enzyme sensor which can measure the subject substances (glucose, urea and so on) in the solution with high accuracy. The method of this embodiment is effective when the enzymes are fixed on a small space, such as on semiconductor chips, whose total length is about several $\mu m$.

It will be further explained in detail referring to the figures as follows.

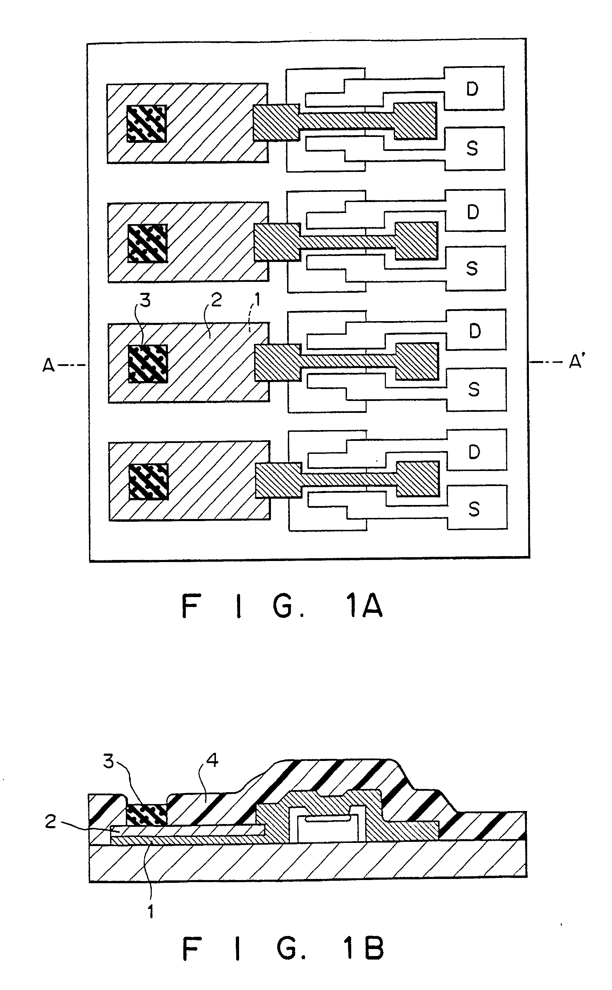

Fig. 1A is a front elevational view of a typical enzyme sensor of The First Embodiment, and Fig. 1B is an on line AA' cross-sectional view of a typical enzyme sensor of The First Embodiment.

The First Embodiment

1. Manufacture of an electrode substrate

Sputtering was performed on an isolation gate 1 of the junction-type FET by Ar plus using a plane parallel plate sputtering apparatus (13-56 Hz), and an $IrO_x$ layer 2 (layer thickness, approximately 800Å; area, 1.8 mm X 5 mm) is then produced. A thickness of 200-5000Å is preferable for the $IrO_x$ layer 2 and a thickness of 500-1500Å is even more preferable, because layer resistance becomes too high when the layer is thin, and the problem of exfoliation or dispersion of the layer coating produced by the lack of uniformity of the layer thickness of the surface layer increases when the layer is thick. 2. Manufacture of an enzyme fixed layer

A portion of the $IrO_x$ layer 2 on the electrode substrate manufactured according to the above procedure 1 is removed and covered with an insulating material (epoxy resin) 4. Twenty ml of pyrrole solution containing glucose oxydase (GOD) is dropped on the open part of the $IrO_x$ layer 2 (5$\mu m$ x 5$\mu m$) and an enzyme fixed layer 3 (5$\mu m$ x 5$\mu m$) is formed after electrolytic polymerization by electrolytic reaction. Thus, the enzyme sensor is produced.

The preparation of an electrolytic solution and electrolysis is performed under the following conditions.

| | |
|---|---|
| pyrrole | 100 mM |
| glucose oxydase | 10 mg/ml |
| NaCl aqueous solution (pH 1.0) | 0.1 M |

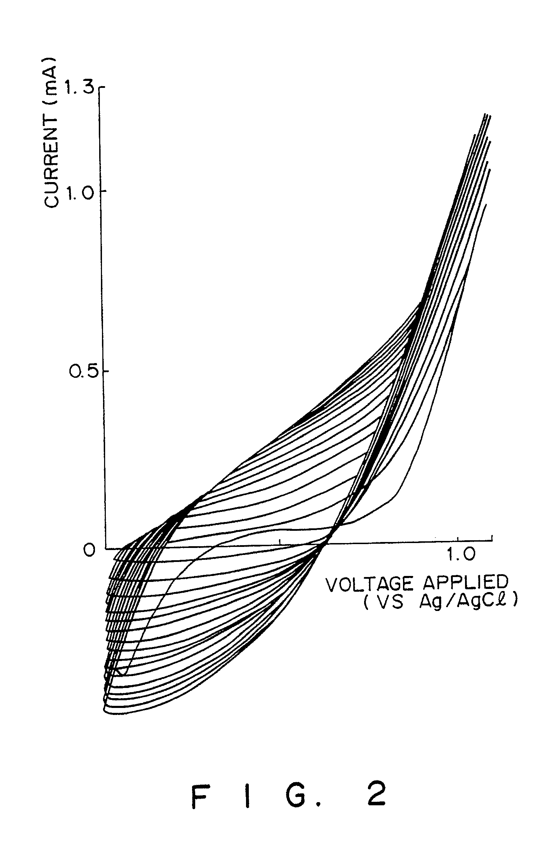

The cyclic voltamogram shown in Fig. 2 is obtained by repeating application on the opening part a number of times and performing the electrolytic reaction for 10 minutes using three electrode cells of the $IrO_x$ layer electrode for a working electrode, Ag/AgCl electrode for a comparative electrode and platinum electrode for a counter electrode. An enzyme fixed layer 2 of a thickness of 1000Å whose GOD is fixed is formed on this $IrO_x$ layer 2.

There are problems that the void of the layer becomes too large when the amount of pyrrole is too small and the sensing speed is slow because of the minuteness of the layer when the amount of pyrrole is too large. In addition, Nernst's characteristics are bad when the amount of GOD is too small.

The First Experimental Embodiment

The response speed for glucose when the enzyme sensor of Embodiment 1 is used as the working electrode and the Ag/AgCl electrode is used as the comparative electrode rose at about 30 mV/hour, and the electric potential difference (-$\Delta E$) at about 95% saturation after about 20 seconds was 10 mV. Thus, it was confirmed that glucose can be measured with high sensitivity. The concentration of glucose at this time was 30 mg/ml. Further, since a decrease of the electric potential difference induced by the elusion of enzymes from the enzyme fixed layer 3 is not observed even if the enzyme sensor is dipped in the glucose coexistence system for several hours, it was confirmed that enzymes are sufficiently fixed.

Comparative Embodiment

When the degree of the thickness of the enzyme fixed layer 3 was changed so as to examine various degrees of thickness, enzyme fixing was not accomplished when the thickness was less than 100Å and confinement of enzymes and a decrease of output response were induced when the thickness was more than 4 μm. Therefore, the electrolytic condition should be adjusted so that the layer thickness of the enzyme fixed layer carrying enzymes become 100Å to 4 μm as described in The First Embodiment.

The Second to Forth Experimental Embodiment

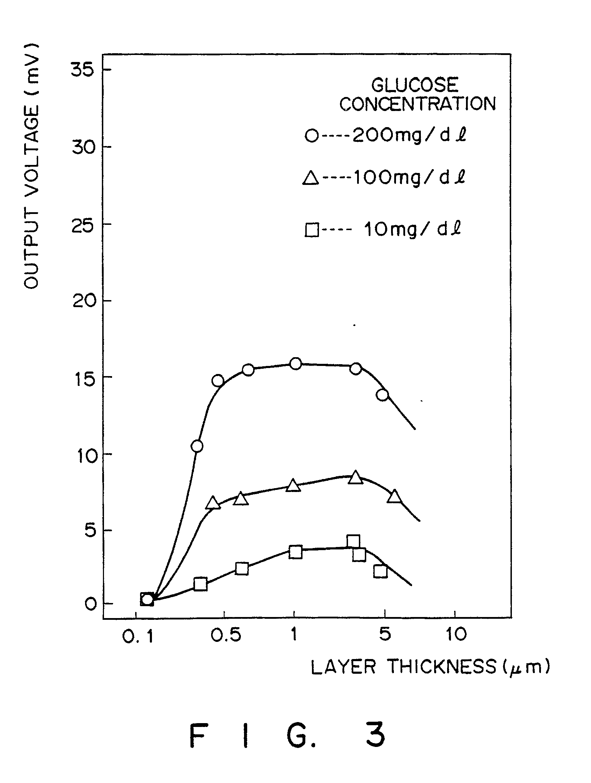

The output voltages were examined under the glucose concentrations of 10 mg/dl (Embodiment 2), 100 mg/dl (The Third Embodiment) and 200 mg/dl (The Forth Embodiment) after changing the layer thickness of the enzyme fixed layer 3 on the $IrO_x$ layer produced in The First Embodiment. As shown in Fig. 3, it is clear that the output voltage hardly changed at a layer thickness of 5000Å to 4 μm. The output voltage lowered when the layer thickness became more than or equal to 4 μm. Therefore, the layer thickness is required to adjust to 5000Å to 4 μm to obtain a sufficient output as the enzyme sensor at such a small space of 5 μm X 5 μm.

The Second and Third Embodiment

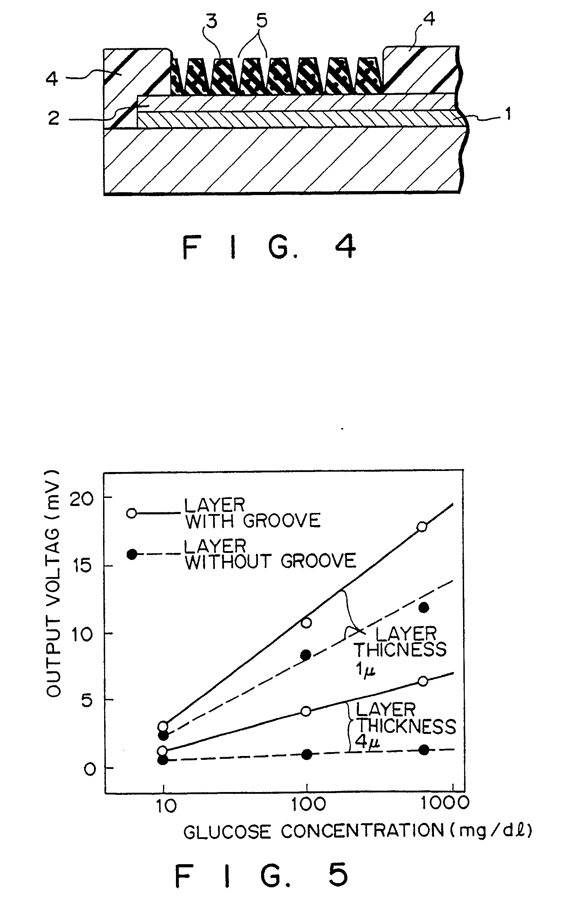

As shown in an enlargement of the sensing part of Fig. 4, the enzyme sensor with the enzyme fixed layer 3 of a thickness of 1 μm (The Second Embodiment) and a thickness of 4 μm (The Forth Embodiment) coated according to the method of The First Embodiment is manufactured. A notch 5 (groove part) is formed on the enzyme fixed layer 3 in order to enlarge the surface area of the layer which reacts with the substrate.

The Fifth Experimental Embodiment

When the output voltage is measured using the enzyme sensor of The Second and Third Embodiments as the work electrode with changing the glucose concentration in the phosphate buffer of pH 6.86, the results shown in Fig. 5 was obtained. The sensitivity increased by forming the groove part. It is supposed that this is caused by the increase of the surface area of the enzyme fixed layer which reacts with the substrate. The shape of this groove part can be rectangle or saw-shape, and its shape is not specified. Alternatively, its surface layer can be increased by forming a porous layer.

As above explained, the enzyme sensor of this invention is an enzyme sensor which the electrically conductive layer is coated on the gate part of the FET and enzymes are further coated cn this layer with the electrolytic layer.

The enzyme sensor has the following characteristics.

1. This enzyme sensor has high sensitivity.
2. The aimed enzymes can be fixed even if the sensor sensing part has a small space of 5 μm X 5 μm.
3. The second electrolytic polymerization can be made by dropping several μl of the pyrrole solution containing enzyme even if enzymes in the fixed layer is inactivated or deteriorated.
4. It is constructed with the MOS-type FET or the isolation gate-type FET of junction-type FET, and can be compacted to the multiple intensive sensor.

The only case of a glucose sensor using glucose oxidase is described in this embodiment, but it is clear that this invention can be applicable to the other enzyme sensor using urease, uricase and so on. Furthermore, the various changes, modifications and supplements of this embodiment can be imparted thereto without departing from the scope of the invention which is limited solely by the appended claims.

**Claims**

4

1. An enzyme sensor comprising an electrically conductive layer and an enzyme fixed layer produced by coating said electrically conductive layer and fixing enzymes wherein:
   a groove part formed on at least some portion of a surface of said enzyme fixed layer.

2. An enzyme sensor according to claim 1 wherein said electrically conductive layer is selected from one of the group of iridium oxide, palladium oxide and ruthenium oxide.

F I G. 1A

F I G. 1B

F I G. 2

F I G. 3

F I G. 4

F I G. 5

**I. CLASSIFICATION OF SUBJECT MATTER** (if several classification symbols apply, indicate all) [6]

According to International Patent Classification (IPC) or to both National Classification and IPC

Int. Cl[4]
G01N27/30

**II. FIELDS SEARCHED**

| Minimum Documentation Searched [7] | |
|---|---|
| Classification System | Classification Symbols |
| IPC | G01N27/30, G01N27/00 |

Documentation Searched other than Minimum Documentation
to the Extent that such Documents are Included in the Fields Searched [8]

| | |
|---|---|
| Jitsuyo Shinan Koho | 1926 – 1989 |
| Kokai Jitsuyo Shinan Koho | 1971 – 1989 |

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| Y | JP, A, 63-66454 (Terumo Corporation) 25 March 1988 (25. 03. 88) (Family : none) | 1, 2 |
| Y | JP, A, 62-185160 (Terumo Corporation ) 13 August 1987 (13. 08. 87) (Family : none) | 1, 2 |
| Y | JP, A, 62-83641 (Sharp Corporation) 17 April 1987 (17. 04. 87) (Family : none) | 1 |

[*] Special categories of cited documents: [10]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance; the claimed invention cannot be considered novel or cannot be considered to involve an inventive step

"Y"  document of particular relevance; the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search | Date of Mailing of this International Search Report |
|---|---|
| July 24, 1989 (24. 07. 89) | August 7, 1989 (07. 08. 89) |

| International Searching Authority | Signature of Authorized Officer |
|---|---|
| Japanese Patent Office | |